# EUROPEAN PATENT APPLICATION

(11) **EP 4 322 175 A1**
(43) Date of publication of application: **14.02.2024**
(21) Application number: 22189479.3
(22) Date of filing: 09.08.2022
(51) Int. Cl.: G16H 30/20, G16H 30/40, G16H 10/60

(54) **A FLEXIBLE METHOD TO REDUCE THE AMOUNT OF DATA TO BE TRANSFERRED BETWEEN DEVICES**

(71) Applicant: Koninklijke Philips N.V., 5656 AG Eindhoven (NL)
(72) Inventor: LUNDT, Bernd, Eindhoven (NL); MAY, Jan Marek, Eindhoven (NL); KOEPNICK, Johannes, 5656AG Eindhoven (NL)
(74) Representative: Philips Intellectual Property & Standards

(57) **Abstract**

In order to reduce the transfer time of medical data, an apparatus for transferring medical data from a clinical-data infrastructure to a cloud-service provider. The apparatus comprises an input unit, a processing unit, and an output unit. The input unit is configured to receive, at the clinical-data infrastructure, medical data to be sent to the cloud-service provider with at least one available cloud service for analyzing the medical data. The processing unit is configured to select a data reduction algorithm from one or more data reduction algorithms based on a data reduction requirement of the at least one available cloud service, and to apply the selected data reduction algorithm to the medical data to generate reduced medical data. The output unit is configured to transmit the reduced medical data to the cloud-service provider.

## Description

### FIELD OF THE INVENTION

The present invention relates to an apparatus and a method for transferring medical data from a clinical-data infrastructure to a cloud-service provider, to a computer program product, and to a computer-readable medium.

### BACKGROUND OF THE INVENTION

Medical imaging is a technique and a process for producing images of a person's internal organs and tissues for use in clinical research and medical intervention. It may also be used to show how certain organs or tissues are functioning. With the use of medical imaging, diseases may be identified and treated as well as interior structures that are covered by the skin and bones. Medical imaging also creates a database of typical anatomy and physiology, which enables the detection of anomalies.

The standard methods to transfer medical data between devices are currently based on the Digital Imaging and Communication in Medicine (DICOM) standard. In case that images should be analyzed, complete images are transferred. In case of X-Ray images this requires a transfer of up to 50 MB of data (e.g., mammography images). If the images shall be analyzed by a cloud service, the transfer from the hospital to the server in the cloud may take a significant part of the total processing time until the result finally arrives at the target device in the hospital again.

### SUMMARY OF THE INVENTION

Thus, there may be a need to reduce the transfer time from clinical devices to cloud services.

The object of the present invention is solved by the subject-matter of the independent claims, wherein further embodiments are incorporated in the dependent claims. It should be noted that the following described aspects of the invention apply also for the apparatus and the method for transferring medical data from a clinical-data infrastructure to a cloud-service provider, the computer program product, and the computer-readable medium.

According to a first aspect of the present invention, there is provided an apparatus for transferring medical data from a clinical-data infrastructure to a cloud-service provider. The apparatus comprises an input unit, a processing unit, and an output unit. The input unit is configured to receive, at the clinical-data infrastructure, medical data to be sent to the cloud-service provider with at least one available cloud service for analyzing the medical data. The processing unit is configured to select a data reduction algorithm from one or more data reduction algorithms based on a data reduction requirement of the at least one available cloud service, and to apply the selected data reduction algorithm to the medical data to generate reduced medical data. The output unit is configured to transmit the reduced medical data to the cloud-service provider.

In other words, the apparatus as described herein receives medical data from a source device at a clinical-data infrastructure. Examples of the source device may include, but are not limited to, Picture Archiving and Communication System (PACS), modality, and viewing station. The medical data may include medical image data acquired with scanning devices, such as computed tomography (CT) and magnetic resonance imaging (MRI). The medical data may comprise DICOM data, Neuroimaging Informatics Technology Initiative (NIfTI) data, and/or data in vendor specific proprietary data formats. The apparatus may analyze the received medical data and check for available cloud service(s) for analyzing the medical data. The available cloud service(s) may comprise one or more data analysis algorithms.

Some data analysis algorithms in the cloud, e.g., some machine learning algorithms, do not need the complete data as input for the analysis. Rather, it is possible to provide a reduced amount of data as input for the analysis. Thus, some data analysis algorithms may have a data reduction requirement, which enables the apparatus to provide a reduced amount of data (instead of the complete data) as input for the analysis. For medical images or videos, the data reduction requirement may include, but are not limited to, reduction of resolution (e.g., downscaling), reduction of size (e.g., excluding a background), reduction of frame rate for videos, and extraction of features.

In one example, some data analysis algorithms in the cloud may not need the complete images as input for the analysis. For example, images acquired with a scanning device (e.g., CT scanner) may be rescaled to a smaller size before feeding them to these data analysis algorithms. Thus, the data reduction requirement of these data analysis algorithms may include rescaling the image data to a predefined size before feeding them to these data analysis algorithms.

In another example, some data analysis algorithms in the cloud may not require the image data itself. Instead, features may be extracted from the image data and fed to these data analysis algorithms. Thus, the data reduction requirement of these data analysis algorithms may include extracting particular features from the image data.

In these cases, the apparatus may select related data reduction algorithms for reducing the amount of data to e.g., the minimum of what is required by the analysis algorithms and transfer the reduced amount of data to the available cloud service(s). The provision of the one or more data reduction algorithms in the apparatus at the clinical-data infrastructure is to avoid transferring irrelevant data to the available cloud service(s). Rather, the apparatus as described herein transfers a relevant subset of data and/or extracted features to the cloud where appropriate data analysis algorithms (e.g., machine learning algorithms) are applied to arrive at an inference. Thus, a significant reduction of the transfer time can be achieved by reducing the amount of data to the minimum of what is required by the analysis algorithms.

In some examples, additional standard compression may be applied which could be lossless compression, or lossy compression depending on the data reduction requirements of the available cloud service(s).

According to examples, the one or more data reduction algorithms may be stored in the cloud, which can be downloaded to the apparatus at the clinical-data infrastructure. *It will be understood that the clinical-data infrastructure may in some cases be confined to a single location, or may be distributed across multiple locations that are geographically separated. In some implementations, the apparatus as described herein may be embodied as, or in, a complete system including hardware and software. In some other implementations, the apparatus may be embodied as a software solution running on an appropriate hardware (e.g., a workstation) provided by the clinical-data infrastructure.*

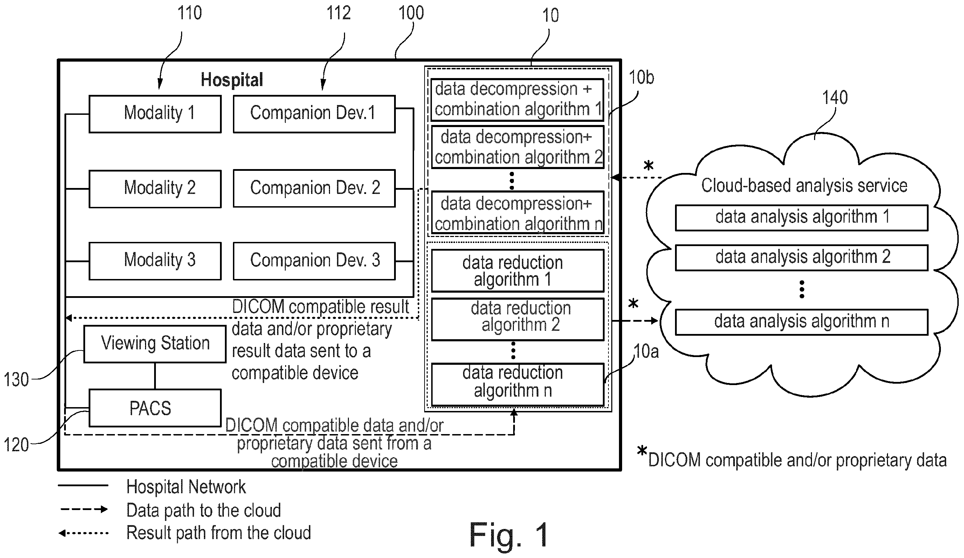

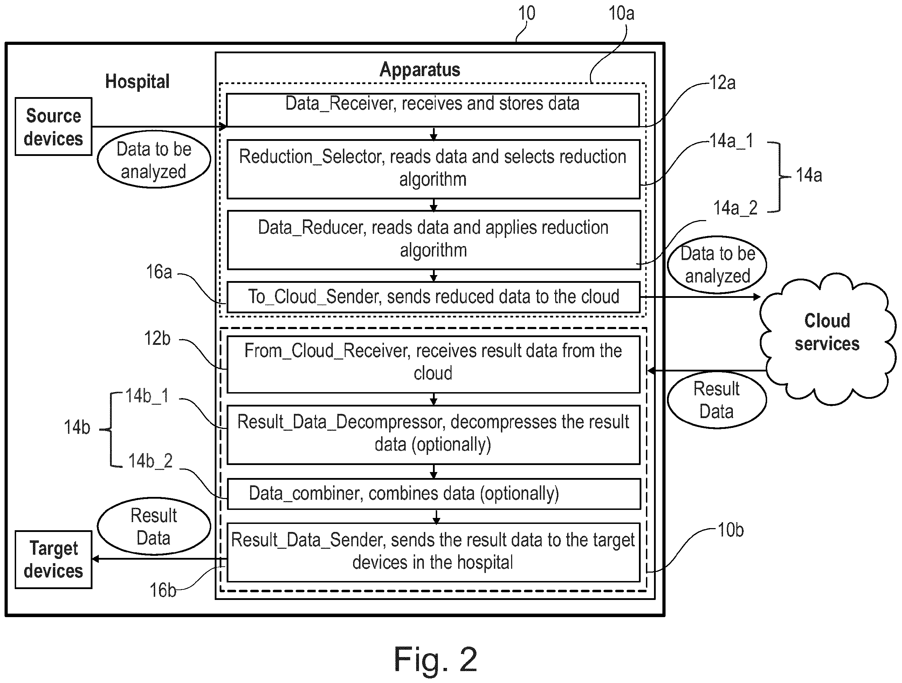

*An exemplary apparatus is illustrated in* *Figs. 1* *and* *2* *and will be described in detail in relation to the example shown in* *Fig. 2**.*

According to an embodiment of the present invention, the medical data comprises one or more of Digital Imaging and Communication in Medicine (DICOM) data, Neuroimaging Informatics Technology Initiative (NIfTI) data, and data in vendor specific proprietary data formats.

According to an embodiment of the present invention, the one or more data reduction algorithms comprise one or more of an algorithm for reducing a resolution of an image and/or a video, an algorithm for reducing a size of an image and/or a video, an algorithm for extracting one or more features from an image and/or a video, and an algorithm for reducing the frame rate of a video.

According to an embodiment of the present invention, the processing unit is further configured to provide identification information of the clinical-data infrastructure to the cloud-service provider.

For example, the apparatus may provide identification information (e.g., customer ID, password, license information, etc.) to connect to the cloud service such that the cloud service recognizes that this connection belongs to a specific clinical-data infrastructure.

*If the cloud-service provider is not the provider* of the *apparatus at the clinical-data infrastructure, the data reduction algorithm(s) cannot be implemented on the apparatus.*

According to an embodiment of the present invention, the processing unit is further configured to receive information describing an availability of a new cloud service for the clinical-data infrastructure.

If the cloud-service provider provides one or more new cloud services, new data reduction/extraction algorithms may be required for the apparatus.

According to an embodiment of the present invention, the processing unit is further configured to update the data reduction algorithm related to the at least one available cloud service.

Accordingly, the data reduction algorithm(s) can be adjusted exactly to the data reduction requirements of the cloud service algorithm.

According to an embodiment of the present invention, the processing unit is configured to receive the information describing an availability of a new cloud service and/or information describing an availability of an update for the one or more data-reduction algorithms by polling a service in the cloud or by an active trigger from the cloud-service provider.

According to an embodiment of the present invention, the input unit is configured to receive result data from the cloud-service provider, wherein the result data is generated by the at least one available cloud service. The output unit is configured to transmit the result data to the clinical-data infrastructure.

In other words, the apparatus may be configured to receive the result from the cloud service(s). The apparatus may provide the result data to one or more target devices at the clinical-data infrastructure, such as modality, PACS, viewing station, and/or a companion device.

This will be described in detail hereinafter and in particular with respect to the example shown in Fig. 2.

According to an embodiment of the present invention, the processing unit is configured to decompress the result data. The transmitted result data is the decompressed result data.

This will be described in detail hereinafter and in particular with respect to the example shown in Fig. 2.

According to an embodiment of the present invention, the processing unit is configured to apply a combination algorithm to combine the received medical data and the result data to create one or more new data objects.

*For example, the new data objects may be a DICOM secondary capture image.*

*In some examples, the medical data may be CT image data, and the result data may be a three-dimensional (3D) volume rendering* of the *CT image data.*

*In some examples, the image data may be X-ray image data, and the result data may be a heat map generated from the X-ray image data.*

*The one or more new data objects may be provided to e.g., a PACS, a viewing station, a modality, and*/*or a companion device (e.g., Tablet PC).*

According to an embodiment of the present invention, the processing unit is configured to download at least one of the data reduction algorithm and the combination algorithm from the cloud.

*In other words, the data reduction algorithm and*/*or the combination algorithm may be stored in the cloud, which can be downloaded to the apparatus at the clinical-data infrastructure.*

According to an embodiment of the present invention, *the processing unit is configured to access the at least one available cloud service through a license owned by the clinical-data infrastructure.*

*For example, the clinical-data infrastructure may have acquired a license to use one or more cloud services. Such a license may either be perpetual, time-limited or usage-limited. In any event, any such a license typically will have other implicit or explicit restrictions regarding the acquirer's rights to cloud services.*

According to a second aspect of the present invention, there is provided a method for transferring medical data from a clinical data infrastructure to a cloud-service provider, the method comprising:
- receiving, at the clinical-data infrastructure, medical data to be sent to the cloud-service provider with at least one available cloud service for analyzing the medical data;
- selecting a data reduction algorithm from one or more data reduction algorithms based on a data reduction requirement of the at least one available cloud service;
- applying the selected data reduction algorithm to the medical data to generate reduced medical data; and
- transmitting the reduced medical data to the cloud-service provider via a communication network.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

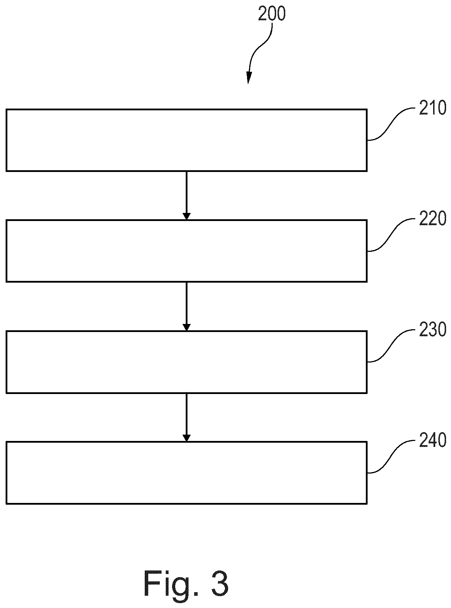

This will be described hereinafter and in particular with respect to the example shown in Fig. 3.

According to another aspect of the present invention, there is provided a computer program product comprising instructions, which when executed by a processing unit, cause the processing unit to carry out the steps of the method according to the second aspect and any associated example. According to a further aspect of the present invention, there is provided a computer-readable data carrier having stored thereon the computer program product.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically illustrates an exemplary clinical-data infrastructure.
Fig. 2 illustrates an exemplary apparatus for transferring the medical data from the clinical-data infrastructure to the cloud-service provider and back.
Fig. 3 illustrates a flowchart describing a method for transferring medical data from a clinical data infrastructure to a cloud-service provider.

### DETAILED DESCRIPTION OF EMBODIMENTS

Fig. 1 schematically illustrates an exemplary clinical-data infrastructure 100 that may be suitable for implementation of the present approach. In the illustrated example, the clinical-data infrastructure 100 comprises one or more medical imaging modalities 110, one or more companion devices 112, and a PACS 120. In the illustrated example, the clinical-data infrastructure 100 is confined to a hospital. It will be understood that the clinical-data infrastructure may be distributed across multiple locations that are geographically separated.

In the illustrated example, the medical imaging modalities 110 comprise Modality 1, Modality 2, and Modality 3 to generate medical image data for a patient (not shown). Although three medical imaging modalities are illustrated in Fig. 1 by way of example, it is to be appreciated that lesser or more medical imaging modalities than the example described herein may be used for certain implementations. The medical imaging modalities 110 may represent any sort of device that generates medical images (e.g., DICOM data, NIfTI data, or any similar image data). Examples of the medical imaging modalities 110 may include, but are not limited to, MRI, ultrasound, medical radiation, angiography, CT, and pathology scanners. The medical imaging modalities 110 may be used in fields including, but not limited to, radiology, cardiology, oncology, nuclear medicine, radiotherapy, neurology, orthopedics, obstetrics, gynecology, ophthalmology, dentistry, maxillofacial surgery, dermatology, pathology, clinical trials, veterinary medicine, and medical/clinical photography.

In the illustrated example, the companion devices 112 comprises companion device 1, companion device 2, and companion device 3. Although three companion devices are illustrated in Fig. 1 by way of example, it is to be appreciated that a lesser or more companion devices than the example described herein may be used for certain implementations. The companion devices 112 may allow a user (e.g., an operator) to interpret and review images acquired by the medical imaging modalities. According to examples, the one or more companion devices 112 may comprise a graphical user interface allowing the user to provide annotations and markups. In some examples, the companion devices 112 may comprise a mobile device, such as a tablet.

In the illustrated example, the PACS 120 is communicatively coupled with the medical imaging modalities 110. The PACS 120 comprises a viewing station 130 for interpreting and reviewing images. Although not shown, the PACS 120 may further comprise other components, such as an acquisition gateway, storage and archiving units, databases, and sophisticated data processors. The universal format for PACS image storage and transfer is DICOM data. Non-image data, such as scanned documents, may be incorporated once encapsulated in the DICOM data.

The clinical-data infrastructure 100 may transfer the medical data, such as DICOM compatible data and/or proprietary data shown in Fig. 1, to a cloud-service provider 140. The cloud-service provider 140 provides a cloud-based analysis service for performing computationally intensive analysis on the medical data. The cloud-based analysis service is a remotely hosted software service that is accessible by means of a local area network (LAN) or wide area network (WAN) such as the internet. The cloud-based analysis service may comprise one or more data analysis algorithms to perform medical image processing, proprietary data analysis, etc. In the example shown in Fig. 1, the one or more data analysis algorithms comprise data analysis algorithm 1, data analysis algorithm 2, ..., data analysis algorithm n, where n is an integer greater or equal to 1. The one or more data analysis algorithms may comprise one or more machine learning algorithms. The one or more machine learning algorithms may be a deep learning algorithm, such as algorithms based on deep neural networks, convolutional deep neural networks, deep belief networks, recurrent neural networks, etc.

The one or more data analysis algorithms in the cloud may be used in a variety of tasks in image analysis including, but not limited to, image segmentation (e.g., atlas-based segmentation), image registration, data visualization, anomaly detection (e.g., anomaly detection in chest in chest radiographs), statistical analysis (e.g., classification, clustering, etc.), etc. In some examples, the one or more data analysis algorithms may comprise a convolutional neural network (CNN) for performing image classification (e.g., classifying and detecting fundus images). In some examples, the one or more data analysis algorithms may comprise a recurrent full convolutional network (RFCN) for performing image segmentation (e.g., performing ventricular segmentation to detect heart diseases in a cardiac MRI image).

As noted above, in the existing method, in case that images should be analyzed, complete images are transferred from the clinical-data infrastructure 100 to the cloud-service provider 140, which may take a significant part of the total processing time until the result finally arrives at the target device in the clinical-data infrastructure again.

Towards this end, as shown in Fig. 1, an apparatus 10 is provided for transferring the medical data from the clinical-data infrastructure 100 to the cloud-service provider 140. The apparatus 10 selects a data reduction algorithm for reducing the amount of data according to the data reduction requirements of the data analysis algorithms. Accordingly, a significant reduction of the transfer time can be achieved by reducing the amount of data to the minimum of what is required by the data analysis algorithms.

In general, the apparatus 10 may comprise various physical and/or logical components for communicating and manipulating information, which may be implemented as hardware components (e.g. computing devices, processors, logic devices), executable computer program instructions (e.g. firmware, software) to be executed by various hardware components, or any combination thereof, as desired for a given set of design parameters or performance constraints. In some implementations, the apparatus 10 may comprise one or more microprocessors or computer processors, which execute appropriate software. The software may have been downloaded and/or stored in a corresponding memory, e.g. a volatile memory such as RAM or a non-volatile memory such as flash. The software may comprise instructions configuring the one or more processors to perform the functions described herein. It is noted that the apparatus 10 may be implemented with or without employing a processor, and also may be implemented as a combination of dedicated hardware to perform some functions and a processor (e.g. one or more programmed microprocessors and associated circuitry) to perform other functions. For example, the at least one processing unit may be implemented in the device or apparatus in the form of programmable logic, e.g. as a Field-Programmable Gate Array (FPGA). Although the apparatus 10 is illustrated as a hardware system by way of example, it will be appreciated that in alternative embodiments, the apparatus 10 may be embodied as a software system (e.g., a software residing in a workstation at the clinical-data infrastructure) that directs hardware to perform the operations. In some implementations, the apparatus 10 may comprise input/output (I/O) units, such as touchscreens, touch panels, touch pads, virtual or regular keyboards, virtual or regular mice, ports, connectors, etc.

Fig. 2 illustrates exemplary components of the apparatus 10 shown in Fig. 1. Although Fig. 2 may show a limited number of components of the apparatus by way of example, it is to be appreciated that a lesser or more components than the example described herein may be preferred for certain implementations. Therefore, the configuration of apparatus 10 may vary from implementation to implementation depending upon numerous factors, such as performance requirements, technological improvements, or other circumstances.

In some examples, as illustrated in Fig. 2, the apparatus 10 may comprise a first module 10a and a second module 10b. The first module 10a is configured to transfer data to be analyzed to the cloud-service provider 140, and the second module 10b is configured to receive result data from the cloud-service provider 140.

The first module 10a comprises a first input unit 12a (shown as Data_Receiver in Fig. 2), a first processing unit 14a, and a first output unit 16a (shown as To_Cloud_Sender in Fig. 2). The first processing unit 14a may comprise a reduction selector 14a_1 and a data reducer 14a_2. Although Fig. 2 may show two separate processing by way of example, in some other implementations (not shown), the reduction selector 14a_1 and the data reducer 14a_2 may be configured as a single processing unit.

The first input unit 12a may be communicatively coupled with one or more source devices to receive medical data to be sent to the cloud-service provider 140. As an example, the first input unit 12a may comprise one or more communication ports, such as USB, Bluetooth, Ethernet, wireless Ethernet, etc., for communicating with the one or more source devices. Examples of the source devices may include, but are not limited to, medical imaging modalities 110, the PACS 120, and the viewing station 130 shown in Fig. 1. The received medical data may comprise DICOM data, NIfTI data, and/or data in vendor specific proprietary data formats. The medical data may include medical images, such as two-dimensional (2D), three-dimensional (3D), and four-dimensional medical images. The medical data may include video data, such as ultrasound video data.

The reduction selector 14a_1 is configured to select a data reduction algorithm from one or more data reduction algorithms based on the data reduction requirement of the at least one available cloud service. The data reducer 14a_2 is configured to apply the selected data reduction algorithm to the medical data to generate reduced medical data.

For example, as illustrated in Fig. 1, the cloud-based analysis service provided by the cloud-service provider 140 may comprise one or more data analysis algorithms, such as data analysis algorithm 1, data analysis algorithm 2,..., data analysis algorithm n. The one or more data analysis algorithms in the cloud may be used for a variety tasks in medical analysis including, but not limited to, image segmentation (e.g., atlas-based segmentation), image registration, data visualization, anomaly detection (e.g., anomaly detection in chest in chest radiographs), statistical analysis (e.g., classification, clustering, etc.), etc.

Some of the data analysis algorithms may not need the complete data as input for the analysis. Therefore, it is possible to provide a reduced amount of data to these data analysis algorithms. For example, as illustrated in Fig. 1, the apparatus 10 may provide one or more data reduction algorithms, such as data reduction algorithm 1, data reduction algorithm 2,...., data reduction algorithm n. For ease of description, the data reduction algorithm 1 is provided to meet the data reduction requirements of data analysis algorithm 1. The data reduction algorithm 2 is provided to meet the data reduction requirements of data analysis algorithm 2. The data reduction algorithm n is provided to meet the data reduction requirements of data analysis algorithm n. However, it will be appreciated that in some other examples (not shown), the number of the data reduction algorithms in the apparatus 10 may be different from the number of the data analysis algorithms in the cloud. For example, two or more data analysis algorithms may use the same data reduction algorithm.

The one or more data reduction algorithms may be provided by the cloud-service provider 140 and stored in the cloud, which may be downloaded to the apparatus 10 at the clinical-data infrastructure 100. For example, the apparatus 10 may access the at least one available cloud service (e.g., data analysis algorithms 1, 2,...n) and the related data reduction algorithms stored in the cloud through a license owned by the clinical-data infrastructure. Such a license may either be perpetual, time-limited or usage-limited.

Some examples of data analysis algorithms and related data reduction algorithms will be described below.

In a first example, the one or more data reduction algorithms may comprise an algorithm for reducing a resolution of an image and/or a video. For example, some data analysis algorithms, such as CNN, may have an enormous GPU memory needs, making it now difficult to fully fit high-resolution MRIs. As a result, depending on the model architecture, 3D medical imaging data with a lower resolution may be fitted into the CNN and other deep learning models. In such case, the apparatus 10 may apply a data reduction algorithm to reduce the resolution of the image data according to the data reduction requirements of the data analysis algorithm (e.g., CNN). The image data with a lower resolution is then transferred to the cloud-based analysis service, and provided as an input for the data analysis algorithm (e.g., CNN).

In a second example, the one or more data reduction algorithms may comprise an algorithm for reducing a size of an image and/or a video. For example, the medical image may comprise an extensive amount of parts labelled as background. For some data analysis algorithms, there may be no (or less) benefit to include the background parts in data processing. Therefore, the apparatus 10 may apply a data reduction algorithm for reducing a size of the image, e.g., by excluding parts that are completely labeled as background, in order to avoid transferring unnecessary data to the cloud-based service.

In a third example, the one or more data reduction algorithms may comprise an algorithm for extracting one or more features from an image and/or a video. A feature extraction is a process through which region of interest (ROI) extracted for analyzing image. For example, a CNN may be used as a classification algorithm for brain tumor classification. The apparatus 10 may receive brain tumor images and apply a data reduction algorithm for extracting one or more features, such as intensity and texture features, from the received brain tumor images. The apparatus 10 then transfers the extracted features, e.g., intensity and texture of the brain tumor images, to the cloud-based service for classification. In these examples, it may be not required to provide the data itself. Instead, extracted features are sufficient to meet the data reduction requirements of the data analysis algorithm provided by the cloud-based analysis service.

In a fourth example, the one or more data reduction algorithms may comprise an algorithm for reducing a frame rate of a video. Taking ultrasound video as an example, a recurrent neural network (RNN) may be used for a variety tasks in medical ultrasound analysis. The RNN may be used to model medical ultrasound video sequences due to the structural characteristic of the network. Depending on the frame rate of the ultrasound video, the RNN may require an enormous GPU memory for medical ultrasound analysis. In such case, the apparatus 10 may apply a data reduction algorithm to reduce the frame rate of the ultrasound video according to the data reduction requirements of data analysis algorithm, and then transfer the video data with a reduced frame rate to the cloud-based service for medical ultrasound analysis.

The one or more data reduction algorithms may be adjusted to the data reduction requirements of the data analysis algorithms in the cloud. If the cloud service provider provides new cloud services, which require new data reduction algorithms, the software on all affected devices in the clinical-data infrastructure should be updated. Thus, in some examples, the first processing unit 14a may be further configured to provide identification information (e.g., license information) of the clinical-data infrastructure to the cloud-service provider. The first processing unit 14a may be further configured to receive information describing an availability of a new cloud service for the clinical-data infrastructure. The first processing unit 14a may be further configured to update the data reduction algorithm related to the at least one available cloud service. The first processing unit 14a may be further configured to receive the information describing an availability of a new cloud service and/or information describing an availability of an update for the one or more data-reduction algorithms by polling a service in the cloud or by an active trigger from the cloud-service provider.

Turning back to Fig. 2, the data reducer 14a_2 provides the reduced data to the first output unit 16a. The first output unit 16a is communicatively coupled with the cloud services over one or more wired and/or wireless networks (e.g., the Internet) to communicate the reduced data to the corresponding data analysis algorithm for performing data analysis.

In the example shown in Fig. 2, the second module 10b comprises a second input unit 12b (shown as From_Cloud_Receiver in Fig. 2), a second processing unit 14b, and a second output unit 16b (shown as Result_Data_Sender in Fig. 2). In the illustrated example, the first and second input units 12a and 12b and the first and second output units 16a and 16b are configured as separated data interfaces. In some other implementations (not shown), some of the input and output units may be configured as one data interface. In addition, although Fig. 2 shows two separate processing units 14a and 14b, in some other implementations (not shown), the first and second processing units 14a and 14b may be configured as a single processing unit.

The second input unit 12b is communicatively coupled with the cloud services over one or more wired and/or wireless networks (e.g., internet) to receive result data from the cloud-service provider 140. The result data is generated by the at least one available cloud service, such as data analysis algorithm 1, data analysis algorithm 2,..., data analysis algorithm n shown in Fig. 1. If the result data comprises compressed data, the second processing unit 14b may decompress the result data, and provide the decompressed result data to the second output unit 16b. In some other examples, no decompression is required.

For example, as illustrated in Fig.2, the second processing unit 14b may comprise a result data decompressor 14b_1 configured to decompress the result data and provide the decompressed result data to the second output unit 16b. The result data decompressor 14b_1 may provide one or more data decompression algorithms, such as data decompression algorithm 1, data decompression algorithm 2,...., data decompression algorithm n shown in Fig. 1. In the illustrated example, the data decompression algorithm 1 is provided to decompress result data obtained from the data analysis algorithm 1. The data decompression algorithm 2 is provided to decompress result data obtained from the data analysis algorithm 2. The data decompression algorithm n is provided to decompress result data obtained from the data analysis algorithm n. However, it will be appreciated that in some other examples (not shown), the number of the data decompression algorithms in the apparatus 10 may be different from the number of the data analysis algorithms in the cloud. For example, one data decompression algorithm may be used to decompress result data obtained from two or more data analysis algorithms. The one or more data decompression algorithms may be provided by the cloud-service provider 140 and stored in the cloud, which may be downloaded to the apparatus 10 at the clinical-data infrastructure 100.

In some examples, as illustrated in Fig. 2, the second processing unit 14b may comprise a data combiner 14b_2 configured to apply a combination algorithm to combine the medical data with the result data to create one or more new data objects and then provide the one or more new data objects to one or more target devices. The data combiner 14b_2 may provide one or more data combination algorithms, such as data combination algorithm 1, data combination algorithm 2,...., data combination algorithm n shown in Fig. 1. In the illustrated example, the data combination algorithm 1 is provided to combine the received medical data with the result data generated by the data analysis algorithm 1. The data combination algorithm 2 is provided to combine the received medical data with the result data generated by the data analysis algorithm 2. The data combination algorithm n is provided to combine the received medical data with the result data generated by the data analysis algorithm n. However, it will be appreciated that in some other examples (not shown), the number of the data combination algorithms in the apparatus 10 may be different from the number of the data analysis algorithms in the cloud. For example, one data combination algorithm may be used to combine the received medical data with the result data generated by two or more data analysis algorithms. The one or more data combination algorithms may be provided by the cloud-service provider 140 and stored in the cloud, which may be downloaded to the apparatus 10 at the clinical-data infrastructure 100.

In one example, the apparatus 10 may receive CT image data from a CT scanner at the clinical-data infrastructure 100, apply a data reduction algorithm to the CT image data, and transfer a reduced amount of data to the cloud-service provider 140. The cloud-service provider 140 may apply a data analysis algorithm to generate e.g., a three-dimensional (3D) volume rendering of the CT data and transfer the 3D volume rendering of the CT data back to the apparatus 10. The data combiner 14b_2 may combine the CT image data with the 3D volume rendering of the CT data to create a new data object, e.g., a DICOM secondary capture image.

In another example, the apparatus 10 may receive X-ray image data from an X-ray imaging system at the clinical-data infrastructure 100, apply a data reduction algorithm to the X-ray image data, and send a reduced amount of data to the cloud-service provider 140. The cloud-service provider 140 may apply a data analysis algorithm to generate a heat map, which is usable to visualize a malignancy or a benign diagnosis associated with a region of interest (ROI), and transfer the heat map back to the apparatus 10. The data combiner 14b_2 may combine the X-ray image data with the received heat map to create a new data object, such as a DICOM secondary capture image.

The second output unit 16b is communicatively coupled with one or more target devices, such as medical imaging modalities 110, the PACS 120, the viewing station 130, the companion devices, and/or any other devices at the clinical-data infrastructure 100, and provides the result data to the one or more target devices.

*Although not shown, it will be appreciated that in some implementations the apparatus may send the medical data to the cloud service through a physical gateway on the same local network as the apparatus. Some or all of the traffic going in and out of that local network can pass through the physical gateway.*

Fig. 3 illustrates a flowchart describing a method 200 for transferring medical data from a clinical data infrastructure to a cloud-service provider. The method 200 may be used in conjunction with the other methods and systems described herein. In particular, the method 200 shown in Fig. 3 may be implemented on the exemplary clinical-data infrastructure 100 shown in Fig. 1 and 2.

At block 210, the method comprises receiving, at the clinical-data infrastructure, medical data to be sent to the cloud-service provider with at least one available cloud service for analyzing the medical data. According to examples, the medical data may comprise DICOM data, NIfTI data, and/or data in vendor specific proprietary data formats.

When the method 200 is implemented on the system shown in Fig. 2, block 210 may be implemented using the first input unit 12a that is communicatively coupled with one or more source devices to receive the medical data.

At block 220, the method comprises selecting a data reduction algorithm from one or more data reduction algorithms based on the data reduction requirement of the at least one available cloud service. According to examples, the one or more data reduction algorithms may be stored in the cloud, which may be downloaded to a local server e.g., based on license information. The one or more data reduction algorithms may be adjusted to the data reduction requirements of the data analysis algorithms in the cloud. If the cloud-service provider provides new cloud services, which require new data reduction algorithms, the software on all affected devices in the clinical-data infrastructure should be updated.

Block 220 may be implemented by the first processing unit 14a, such as the reduction selector 14a_1 of the first processing unit 14a shown in Fig. 2. In Fig. 2, the first processing unit 14a may provide identification information (e.g., customer ID, password, etc.) of the clinical-data infrastructure to the cloud-service provider such that the cloud service recognizes that this connection belongs to a specific clinical-data infrastructure. *If the cloud-service provider is not the provider of the apparatus at the clinical-data infrastructure, the data reduction algorithm(s) cannot be implemented on the apparatus..* The first processing unit 14a may be configured to receive information describing an availability of a new cloud service for the clinical-data infrastructure. The first processing unit 14a may update the data reduction algorithm related to the at least one available cloud service, e.g., if the cloud servicer provider changes model infrastructure of a data analysis algorithm, which require new data reduction algorithms. The information about updates available and/or the new cloud service may be triggerable by a software running in the cloud or by polling a server in the cloud. For example, the first processing unit 14a may be configured to receive the information describing an availability of a new cloud service and/or information describing an availability of an update for the one or more data-reduction algorithms by polling a service in the cloud or by an active trigger from the cloud-service provider.

Examples of the data reduction algorithms may include, but are not limited to, an algorithm for reducing a resolution of an image and/or a video, an algorithm for reducing a size of an image and/or a video, an algorithm for extracting one or more features from an image and/or a video, and an algorithm for reducing a frame rate of a video.

At block 230, the method 200 comprises applying the selected data reduction algorithm to the medical data to generate reduced medical data. This may be implemented by the first processing unit 14a, such as the data reducer 14a_2 of the first processing unit 14a shown in Fig. 2. As described in relation to Fig. 2, the data reducer 14a_2 of the first processing unit 14a may generate reduced medical data including, but not limited to, reduction of resolution, reduction of size, reduction of frame rate, and extraction of features.

At block 240, the method 200 comprises transmitting the reduced medical data to the cloud-service provider via a communication network. Block 240 may be implemented by the first output unit 16a shown in Fig. 2, which is communicatively coupled with the cloud-service provider 140 over one or more wired and/or wireless networks (e.g., the Internet) to communicate the reduced amount of data to the cloud services.

With the method 200 as described herein, a significant reduction of the transfer time can be achieved by reducing the amount of data to the minimum of what is required by the analysis algorithms.

According to examples (not shown), the method may further comprise receiving result data from the cloud-service provider and transmitting the result data to the clinical-data infrastructure. The result data is generated by the at least one available cloud service. This may be implemented by the second input unit 12b and the second output unit 16b shown in Fig. 2. As described in relation to Fig. 2, the second input unit 12b is communicatively coupled with the cloud-service provider over one or more wired and/or wireless communication networks to receive the result data. In some examples, the result data may be decompressed by the result data decompressor 14b_1. In some examples, no decompression is required for the result data. The result data (or decompressed result data) may be provided to one or more target devices, such as imaging modalities 110, the PACS 120, the viewing station 130, and other devices at the clinical-data infrastructure 100 shown in Fig. 1.

According to examples (not shown), the method may further comprise applying a combination algorithm to combine the received medical data and the result data to create one or more new data objects, such as DICOM secondary capture image. The combination algorithm may be provided by the cloud-service provider and stored in the cloud, which may be downloaded to a local server at the clinical-data infrastructure. This may be implemented by the data combiner 14b_2 shown in Fig. 2. The one or more new data objects may be provided to one or more target devices, such as imaging modalities 110, the PACS 120, the viewing station 130, and other devices at the clinical-data infrastructure 100 shown in Fig. 1.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

In the claims, the word "comprising" does not exclude other elements or steps, and the indefinite article "a" or "an" does not exclude a plurality.

It should be noted that the terms "first", "second", and the like (if existent) in the embodiments of this application are intended to distinguish between similar objects but do not necessarily indicate a specific order or sequence.

## Claims

1. An apparatus (10) for transferring medical data from a clinical-data infrastructure to a cloud-service provider, the apparatus comprising:
- an input unit (12a) configured to receive, at the clinical-data infrastructure, medical data to be sent to the cloud-service provider with at least one available cloud service for analyzing the medical data;
- a processing unit (14a) configured to select a data reduction algorithm from one or more data reduction algorithms based on a data reduction requirement of the at least one available cloud service, and to apply the selected data reduction algorithm to the medical data to generate reduced medical data; and
- an output unit (16a) configured to transmit the reduced medical data to the cloud-service provider.

2. The apparatus according to claim 1,
wherein the medical data comprises one or more of:
- Digital Imaging and Communication in Medicine, DICOM, data;
- Neuroimaging Informatics Technology Initiative, NIfTI, data; and
- Data in vendor specific proprietary data formats.

3. The apparatus according to claim 1 or 2,
wherein the one or more data reduction algorithms comprise one or more of:
- an algorithm for reducing a resolution of an image and/or a video;
- an algorithm for reducing a size of an image and/or a video;
- an algorithm for extracting one or more features from an image and/or a video; and
- an algorithm for reducing a frame rate of a video.

4. The apparatus according to any one of the preceding claims,
wherein the processing unit is further configured to provide identification information of the clinical-data infrastructure to the cloud-service provider.

5. The apparatus according to any one of the preceding claims,
wherein the processing unit is further configured to receive information describing an availability of a new cloud service for the clinical-data infrastructure.

6. The apparatus according to any one of the preceding claims,
wherein the processing unit is further configured to update the one or more data reduction algorithms related to the at least one available cloud service.

7. The apparatus according to claim 5 or 6,
wherein the processing unit is configured to receive the information describing an availability of a new cloud service and/or information describing an availability of an update for the one or more data-reduction algorithms by polling a service in the cloud or by an active trigger from the cloud-service provider.

8. The apparatus according to any one of the preceding claims,
wherein the input unit is configured to receive result data from the cloud-service provider, wherein the result data is generated by the at least one available cloud service; and
wherein the output unit is configured to transmit the result data to the clinical-data infrastructure.

9. The apparatus according to claim 8,
wherein the processing unit is configured to decompress the result data; and
wherein the transmitted result data is the decompressed result data.

10. The apparatus according to claim 8 or 9,
wherein the processing unit is configured to apply a combination algorithm to combine the received medical data and the result data to create one or more new data objects.

11. The apparatus according to any one of the claims,
wherein the processing unit is configured to download at least one of the data reduction algorithm and the combination algorithm from the cloud.

12. The apparatus according to any one of the preceding claims,
wherein the processing unit is configured to access the at least one available cloud service through a license owned by the clinical-data infrastructure*.*

13. A method (200) for transferring medical data from a clinical data infrastructure to a cloud-service provider, the method comprising:
- receiving (210), at the clinical-data infrastructure, medical data to be sent to the cloud-service provider with at least one available cloud service for analyzing the medical data;
- selecting (220) a data reduction algorithm from one or more data reduction algorithms based on a data reduction requirement of the at least one available cloud service;
- applying (230) the selected data reduction algorithm to the medical data to generate reduced medical data; and
- transmitting (240) the reduced medical data to the cloud-service provider via a communication network.

14. A computer program product comprising instructions, which when executed by a processing unit, cause the processing unit to carry out the steps of the method of claim 13.

15. A computer-readable data carrier having stored thereon the computer program product of claim 14.
